# EUROPÄISCHE PATENTSCHRIFT

(11) **EP 3 245 931 B1**
(45) Veröffentlichungstag und Bekanntmachung des Hinweises auf die Patenterteilung: **12.12.2018**
(21) Anmeldenummer: 16170109.9
(22) Anmeldetag: 18.05.2016
(51) Int. Cl.: A61B 1/00, A61B 90/30, G02B 6/36

(54) **MAGNETISCHE LICHTLEITERSTECKVERBINDUNG**
MAGNETIC LIGHT CONDUCTOR PLUG CONNECTION
CONNECTEUR MAGNETIQUE A CONDUITS DE LUMIERE

(43) Veröffentlichungstag der Anmeldung: 22.11.2017
(73) Patentinhaber: Oertli-Instrumente AG, 9442 Berneck (CH)
(72) Erfinder: SANSEVERINO, Flavio, 9434 Au (CH); BRILL, Norbert, 8280 Kreuzlingen (CH)
(74) Vertreter: Frischknecht, Harry Ralph

(56) Entgegenhaltungen:
- EP-A1- 2 975 440
- JP-A- 2005 092 082
- US-A1- 2007 191 823
- US-A1- 2009 275 930
- US-A1- 2016 087 726

## Beschreibung

### TECHNISCHES GEBIET

Die vorliegende Erfindung betrifft ein ophthalmisches Lichtinstrument nach dem Oberbegriff von Anspruch 1 sowie ein ophthalmisches Beleuchtungssystem umfassend solch ein Lichtinstrument nach dem Oberbegriff von Anspruch 7.

### STAND DER TECHNIK

In der Augenchirurgie ist eine ausreichende Beleuchtung des Augeninneren bzw. des Augenhintergrundes von grosser Bedeutung. Das menschliche Auge reagiert dabei sehr empfindlich auf blaues, violettes oder UV-Licht und wird bei zu hoher Lichtintensität geschädigt. Ist die Lichtintensität zu hoch oder wird eine bestimmte Lichtdosis überschritten, kann das Auge innerhalb kurzer Zeit thermisch oder photochemisch geschädigt werden. Eingesetzt werden in der Augenchirurgie oftmals faseroptische Lichtleiter für die Endoillumination, welche durch eine kleine Inzision in der "pars plana" ins Auge eingeführt werden.

Aus der WO 2012/154435 A1 ist ein beleuchtetes mikrochirurgisches Instrument bekannt, welches über eine optische Glasfaser mit abgeschrägter Endfläche verfügt. Dabei ist der Refraktionsindex der optischen Faser derart grösser als der Refraktionsindex des Auges, dass eine winklige Verteilung des emittierten Lichtstrahls im Auge erfolgt.

Eine beleuchtete Lasersonde mit verstellbarem Beleuchtungsbereich ist aus der WO 2005/048817 A2 bekannt, wobei ein Mechanismus mit der Lasersonde wirkverbunden ist, um die Laserfaser zwischen einer zurückgezogenen Position und einer ausgestreckten Position zu bewegen.

Aus der US 2009/275930 A1 ist eine Einwegspitze mit einer optischen Faser bekannt, welche lösbar mit einer laserchirurgischen Vorrichtung verbunden werden kann. Dazu kann die Vorrichtung einen Magneten aufweisen und die Spitze kann mit einem magnetischen Gehäuse in Verbindung stehen.

Allerdings beleuchten Lichtleiter resp. Lichtsonden den intraokularen Raum oft unzureichend, so dass mehrere Lichtsonden eingesetzt, weitere Lichtleiter verwendet, oder während der Operation die Lichtsonden umgesetzt werden müssen. Für den Patienten kann dies nachteilig sein, weil dadurch die Operation länger dauert oder möglicherweise nicht so präzise durchgeführt werden kann. Zudem kann die Kopplung der Lichtsonden bzw. Lichtleitern mit einer Lichtquelle einen komplizierten oder aufwändigen Kopplungsmechanismus benötigen, bei welchem das Operationspersonal z.B. manuell eine Fixierung mittels einer Rastverbindung vornehmen muss. Eine weniger aufwändige Kopplung kann beispielsweise durch Verklemmen der Lichtsonde bzw. Lichtleiter mit der Lichtquelle erzeugt werden, wobei also keine feste Fixierung sondern ein Anschlag erfolgt. Hierbei besteht allerdings die Gefahr, dass die Kopplung nicht ausreichend ist und sich die Lichtsonde bzw. der Lichtleiter verschiebt oder sogar von der Lichtquelle löst.

### DARSTELLUNG DER ERFINDUNG

Aufgabe der Erfindung ist es daher ein ophthalmisches Lichtinstrument bzw. ein ophthalmisches Beleuchtungssystem anzugeben, welches die Nachteile des Standes der Technik überwindet. Insbesondere soll das ophthalmische Lichtinstrument bzw. -system eine verbesserte Kopplung mit einer Lichtquelle ermöglichen.

Erfindungsgemäss wird diese Aufgabe durch ein opthalmisches Lichtinstrument gemäss Anspruch 1 bzw. durch ein ophthalmisches Beleuchtungssystem gemäss Anspruch 7 gelöst. Weitere Ausführungsformen sind in den abhängigen Ansprüchen angegeben.

In einem ersten Aspekt wird also ein opthalmisches Lichtinstrument angegeben, welches einen Stecker des Lichtinstruments mit einer Anschlagsfläche und einen am Stecker des Lichtinstruments gelagerten Lichtleiter zum Zuleiten von Licht bzw. eines Lichtstrahls an eine chirurgische Stelle im Auge umfasst, wobei der Lichtleiter ein proximales Ende zum Einkoppeln von Licht von einer Lichtquelle sowie ein distales Ende zum Emittieren des Lichts aufweist. Der Stecker des Lichtinstruments ist magnetisch anziehbar bzw. anziehend ausgebildet und über seine Anschlagsfläche derart zur Lichtquelle positionierbar und zur Lichtquelle abnehmbar verbindbar, dass das proximale Ende des Lichtleiters im Brennpunkt der Lichtquelle zu liegen kommt, so dass eine Einkopplung des Lichts in das Lichtinstrument im Brennpunkt der Lichtquelle erfolgt.

Während den andauernden Manipulationen durch einen Operateur kann der Stecker des Lichtinstruments durch die über den Lichtleiter übertragene Zugspannung aus dem Brennpunkt gezogen werden, was zu einer verminderten oder schlimmstenfalls sogar unterbrochenen Beleuchtung am chirurgischen Wirkungsort im Auge führt. Indem der Stecker des Lichtinstruments magnetisch anziehbar bzw. anziehend ausgebildet ist und somit eine magnetische Anziehung erfolgt, korrigiert beziehungsweise verhindert die magnetische Anziehung dieses Verhalten. Das Lichtinstrument lässt sich somit aufgrund der magnetischen Anziehung einfach und zuverlässig mit einer Lichtquelle koppeln.

Bei Einwirken einer Zugspannung auf den Stecker des Lichtinstruments kann dieser von der Lichtquelle weg bewegt werden. Durch die magnetische anziehbare bzw. anziehende Ausbildung wird der Stecker des Lichtinstruments nach dem Einwirken der Zugspannung auf das Lichtinstrument somit mit seiner Anschlagsfläche wieder derart zur Lichtquelle positioniert beziehungsweise gehalten, dass die Einkopplung des Lichts in das Lichtinstrument im Brennpunkt der Lichtquelle erfolgt. Somit liegt die Einkoppelseite des Lichtinstrumentes immer im Brennpunkt der Lichtquelle, was für eine maximale Lichtleistung / Beleuchtung sorgt sowie eine stabile und reproduzierbare Lichtleistung des Lichtinstrumentes gewährleistet. Das Lichtinstrument sorgt somit für verbesserte und zuverlässige Beleuchtungsverhältnisse am chirurgischen Wirkungsort im Auge.

Zudem erlaubt der magnetisch anziehende bzw. anziehbare Stecker des Lichtinstruments eine einfache Positionierung und abnehmbare Verbindung zur Lichtquelle, da dazu keine Verriegelungsmechanismen wie z.B. Schraub- oder Bajonettverschlüsse betätigt werden müssen und aufgrund der magnetischen Kraft nur ein geringer Kraftaufwand des Operateurs für die Kopplung mit der Lichtquelle erforderlich ist. Das Lichtinstrument verfügt somit über eine einfache Handhabung.

Das Lichtinstrument kann in einer bevorzugten Variante ein Beleuchtungselement sein, welches ausschliesslich für die Beleuchtung vorgesehen ist. Alternativ kann das Lichtinstrument aber auch einen integralen Teil eines beleuchteten Instrumentes sein, wie beispielsweise eines beleuchteten Cutters, einer beleuchteten Lasersonde, einer beleuchteten Pinzette, etc.

Die Ausdrucksweise, dass der Lichtleiter am Stecker des Lichtinstruments gelagert ist, schliesst sämtliche Arten der Lagerung des Lichtleiters am Stecker des Lichtinstruments ein. Beispielsweise kann der Lichtleiter im Inneren des Steckers des Lichtinstruments oder an einer Aussenfläche des Steckers des Lichtinstruments gelagert sein.

Vorzugsweise ist der Lichtleiter fest, das heisst nicht zum Stecker des Lichtinstruments verschieblich, gelagert. Der Lichtleiter könnte beispielsweise am Stecker des Lichtinstruments stoffschlüssig über eine Klebeverbindung gelagert sein.

Alternativ kann der Lichtleiter in einem Führungsrohr fest gelagert sein, welches sich durch den Stecker hindurch erstreckt und welches fest mit dem Stecker in Verbindung steht. Das heisst, der Lichtleiter ist über das Führungsrohr mit dem Stecker in Verbindung. Das Führungsrohr umgibt den Lichtleiter hülsenartig. Das Führungsrohr kann beispielsweise aus Metall oder Kunststoff sein. Vorzugsweise erstreckt sich das Führungsrohr mit seinem proximalen Ende aus dem Stecker des Lichtinstrumentes hinaus, so dass das Führungsrohr in eine Buchse einschiebbar ist. Auch kann das distale Ende des Führungsrohrs aus dem Innenraum des Steckers hinaus ragen.

Am distalen Ende des Lichtleiters umfasst der Lichtleiter vorzugsweise ein Handgriff, welcher vom Operationspersonal ergriffen werden kann.

Magnetismus ist ein physikalisches Phänomen, das sich unter anderem als Kraftwirkung zwischen Magneten, magnetisierten bzw. magnetisierbaren Gegenständen und bewegten elektrischen Ladungen äussert. Die Vermittlung dieser Kraft erfolgt über ein Magnetfeld, das einerseits von diesen Objekten erzeugt wird und andererseits auf sie wirkt. Diamagnetismus, Paramagnetismus und Ferromagnetismus bezeichnen unterschiedliche magnetische Eigenschaften der Materie. Ein ferromagnetischer Stoff kann von einem Magneten stark angezogen werden. Ein paramagnetisches Material kann dagegen nur sehr schwach angezogen und ein diamagnetischer Stoff kann sogar schwach abgestossen werden.

Die magnetischen Eigenschaften der Materie werden durch Elementarmagnete erklärt, wobei es sich überwiegend um Elektronenspins oder Kernspins handelt, die als atomare Spins eine magnetische Wirkung besitzen. Die magnetischen Kräfte von Permanentmagneten oder Dauermagneten werden darüber erklärt, dass die Elementarmagnete an den einzelnen Atomen des Materials parallel ausgerichtet sind. Diese parallele Ausrichtung der atomaren Spins findet sich in ferromagnetischen Stoffen. Die Ausrichtung der Elementarmagnete kann beispielsweise durch Zuführen von thermischer Energie oder durch Anlegen eines äusseren entgegengesetzt gerichteten Magnetfeldes zerstört werden. Dies bezeichnet man als Entmagnetisierung.

Unter "magnetisch anziehbar" und "magnetisch anziehend" wird hierbei verstanden, dass der magnetisch anziehbare Materialanteil vom magnetisch anziehenden Materialanteil aufgrund der magnetischen Anziehungskraft angezogen wird.

Der Stecker des Lichtinstruments weist in einer Variante einen magnetisierbaren Materialanteil oder einen magnetisierten Materialanteil auf. Hierbei kann der Stecker des Lichtinstruments mit einem magnetisch anziehbaren Materialanteil auf Seiten der Lichtquelle zusammenarbeiten. In einer anderen Variante weist der Stecker des Lichtinstruments einen magnetisch anziehbaren Materialanteil auf. Hierbei kann der Stecker des Lichtinstruments mit einem magnetisierbaren oder einem magnetisierten Materialanteil auf Seiten der Lichtquelle zusammenarbeiten.

Hierbei gilt es zu verstehen, dass unter "Materialanteil" einen Anteil an magnetisierbarem bzw. magnetisiertem bzw. magnetischem Material bezüglich dem restlichen Material gemeint ist, welcher Anteil vergleichsweise wenig, d.h. im Wesentlichen keinen Anteil an magnetisierbarem bzw. magnetisiertem bzw. magnetischem Material, oder viel, d.h. einen grossen Anteil an magnetisierbarem bzw. magnetisiertem bzw. magnetischem Material, oder alles, d.h. vollständig aus magnetisierbarem bzw. magnetisiertem bzw. magnetischem Material, sein kann.

Der magnetisierbare Materialanteil im Stecker des Lichtinstruments kann magnetisiert sein.

Wird ein Material einem äusseren Magnetfeld ausgesetzt, so kommt es zu einer Magnetisierung des Materials. Die Richtung und Stärke dieser Magnetisierung beruht dabei auf intrinsischen Eigenschaften des Materials, so dass die Magnetisierung von Materie in einem äusseren Feld, also die Ausrichtung der Elementarmagnete im Material, dem äusseren Magnetfeld gleichgerichtet oder entgegengerichtet sind. Ein ferromagnetischer Stoff wird in einem äusseren Magnetfeld selbst magnetisiert. Auch nicht ferromagnetische Stoffe wie z.B. Dia- und Paramagnete lassen sich magnetisieren, allerdings ist der Effekt in diesen Materialien deutlich schwächer. Bei Dia- und Paramagneten verschwindet zudem die Magnetisierung wieder, wenn das äussere Magnetfeld abgeschaltet wird, nicht aber bei ferromagnetischen Stoffen.

Der magnetisierbare Materialanteil und/oder der magnetisierte Materialanteil kann aus ferrimagnetischem Material bestehen, und/oder der magnetisierbare Materialanteil und/oder der magnetisierte Materialanteil können aus ferromagnetischem Material bestehen.

Ein Material wird ferromagnetisch genannt, wenn es in einem externen Magnetfeld eine eigene, vom äusseren Magnetfeld unabhängige, sogenannte spontane Magnetisierung zeigt. Das externe Magnetfeld bestimmt die Richtung der Elementarmagnete, während deren Betrag davon unabhängig ist. Davon unterscheidet sich der Ferrimagnetismus, bei welchem die Elementarmagnete jeweils abwechselnd entgegengesetzt gerichtet und in den zwei Richtungen unterschiedlich stark ausgeprägt sind, weshalb für jedes Paar eine Magnetisierung verbleibt. Das makroskopische Verhalten von ferrimagnetischen Materialien ist somit eine schwächere Form des Ferromagnetismus.

Der magnetisierbare Materialanteil und/oder der magnetisierte Materialanteil und/oder der magnetisch anziehende Materialanteil ist partikelförmig ausgebildet und wird in einer Kunststoffmatrix eingebettet. Der Stecker des Lichtinstruments ist also im Wesentlichen aus Kunststoff mit eingebetteten magnetisierbaren oder magnetisierten Partikeln hergestellt. Die Kunststoffmatrix wird vorzugsweise durch Spritzgiessen oder Extrudieren hergestellt, wobei die Partikel während des Spritzgiessens bzw. des Extrudierens einbettbar sind.

Der magnetisierbare Materialanteil kann während des Spritzgiessens oder während des Extrudierens durch Anlegen eines Magnetfeldes magnetisiert werden.

So kann also beispielsweise der Stecker des Lichtinstruments aus Ferritpartikel, welche in Kunststoff gebunden sind, hergestellt werden, wobei die Ferritpartikel bereits magnetisiert oder nicht magnetisiert sind oder wobei die Ferritpartikel während dem Herstellungsprozess einem äusseren Magnetfeld ausgesetzt und dadurch erst magnetisiert werden.

Die Herstellung des Steckers des Lichtinstruments als Spritzguss- bzw. Extrusionssteil erlaubt eine kostengünstige Herstellung in grossen Stückzahlen. Insbesondere eignet sich der Stecker des Lichtinstruments somit als Einwegteil, was vor allem im Hinblick auf die Anforderungen betreffend Hygiene und Sterilität eines Operationssaales von grosser Bedeutung ist.

Der magnetisch anziehbare und partikelförmige Materialanteil enthält z.B. Eisen, Nickel und / oder Kobald. Daneben kann der magnetisch anziehbare und partikelförmige Materialanteil aus Legierungen (z.B. Samarium - Kobalt) oder Neodym -Eisen Bohr) bestehen.

Der Stecker des Lichtinstruments kann dabei vollständig aus dem magnetisierbaren Materialanteil oder aus dem magnetisierten Materialanteil bestehen.

Es ist aber auch denkbar, dass der magnetisierbare Materialanteil oder der magnetisierte Materialanteil im Bereich des proximalen Endes des Steckers des Lichtinstruments, insbesondere in der Anschlagsfläche des Steckers des Lichtinstruments, angeordnet ist.

In anderen Worten kann der magnetisierbare Materialanteil bzw. der magnetisierte Materialanteil wie beispielsweise Ferritpartikel nur bereichsweise in ein Kunststoffmaterial eingebunden werden.

Auch denkbar ist, dass der magnetisierte Materialanteil als mindestens ein Magnet.

Das heisst, dass also anstelle oder zusätzlich zu dem magnetisierbaren bzw. magnetisierten partikelförmigen Materialanteil ein Dauermagnet, oder auch Permanentmagnet genannt, und / oder eine Magnetspule in den Stecker des Lichtinstruments eingebracht oder an diesem angebracht wird. Beispielsweise könnte solch ein Dauermagnet bzw. Magnetspule im proximalen Bereich des Steckers des Lichtinstruments, z.B. in der Anschlagsfläche des Steckers des Lichtinstruments angebracht oder eingebracht werden.

Der Stecker des Lichtinstruments weist vorzugsweise einen im Wesentlichen zylinderförmigen Körper auf, welcher im Inneren einen Hohlraum definiert. Der Hohlraum mündet bevorzugterweise in eine Eintrittsöffnung am proximalen Ende und in eine Austrittsöffnung am distalen Ende des Steckers des Lichtinstruments, wobei sich der Lichtleiter durch die Eintrittsöffnung via den Hohlraum durch die Austrittsöffnung hindurch erstreckt.

Es ist bevorzugt, dass der Lichtleiter dabei im Inneren des Steckers des Lichtinstruments, insbesondere im Bereich der Eintritts- und Austrittsöffnung des Steckers des Lichtinstruments, fest gelagert ist

Vorzugsweise ist die Anschlagsfläche am proximalen Ende des Steckers des Lichtinstruments als einen Flansch ausgebildet, dessen Durchmesser grösser als der Durchmesser des im Wesentlichen zylinderförmigen Körpers des Steckers des Lichtinstruments ist.

Es ist allerdings auch denkbar, dass der Flansch und der im Wesentlichen zylinderförmige Körper des Steckers des Lichtinstruments denselben Durchmesser aufweisen, dass also die Anschlagsfläche zusammen mit dem Köper einen Stecker des Lichtinstruments in Zylindergestalt mit konstantem Durchmesser bilden.

Es ist dabei bevorzugt, dass der im Wesentlichen zylinderförmige Körper des Steckers des Lichtinstruments und die als Flansch ausgebildete Anschlagsfläche einstückig ausgebildet sind und beispielsweise im Herstellungsverfahren durch Spritzgiessen oder Extrusion als ein einzelnes Spritzguss- bzw. Extrusionssteil hergestellt werden.

Vorzugsweise weist der Hohlraum zwei Abschnitte unterschiedlichen Durchmessers auf. In einem ersten Abschnitt weist der Hohlraum einen Durchmesser auf, der im Wesentlichen dem Aussendurchmesser des Lichtleiters entspricht. In diesem ersten Abschnitt ist der Lichtleiter im Hohlraum gelagert. In einem zweiten Abschnitt, welcher sich dem ersten Abschnitt in anschliesst, weist der Hohlraum einen Durchmesser auf, welcher grösser ist als der erste Abschnitt. Die vergösserte Ausbildung hat den Vorteil, dass ein Knicken des Lichtleiters vermieden werden kann.

In anderen Worten schliesst sich der zweite Abschnitt in distaler Richtung gesehen, also ausgehend von der Lichtquelle in Richtung des Steckers des Lichtinstruments, distal an den ersten Abschnitt an. Der erste Abschnitt des Hohlraums mündet somit in die Eintrittsöffnung am proximalen Ende des Steckers des Lichtinstruments, während der zweite Abschnitt des Hohlraums in die Austrittsöffnung am distalen Ende des Steckers des Lichtinstruments mündet.

In einem weiteren Aspekt wird ein ophthalmisches Beleuchtungssystem umfassend ein Lichtinstrument wie oben beschrieben und eine Buchse der Lichtquelle mit einer weiteren Anschlagsfläche angegeben. Die Buchse der Lichtquelle ist, insbesondere korrespondierend zum Stecker des Lichtinstruments, magnetisch anziehbar bzw. anziehend ausgebildet. Die Anschlagsfläche des Steckers des Lichtinstruments kommt mit der weiteren Anschlagsfläche der Buchse der Lichtquelle in Kontakt, so dass die Buchse der Lichtquelle derart zur Lichtquelle positionierbar und mit dem Stecker des Lichtinstruments abnehmbar verbindbar ist, dass das proximale Ende des Lichtleiters im Brennpunkt der Lichtquelle zu liegen kommt, so dass eine Einkopplung des Lichts in das Lichtinstrument im Brennpunkt der Lichtquelle erfolgt.

Indem der Stecker des Lichtinstruments magnetisch anziehbar bzw. anziehend ausgebildet ist und die Buchse der Lichtquelle magnetisch anziehend bzw. anziehbar ausgebildet ist, gewährleistet die magnetische Anziehungskraft zwischen dem Stecker des Lichtinstruments und der Buchse der Lichtquelle, dass der Stecker des Lichtinstruments immer mit seiner Anschlagsfläche auf der weiteren Anschlagsfläche der Buchse der Lichtquelle aufliegt und sich das Beleuchtungssystem somit einfach und zuverlässig bezüglich Buchse der Lichtquelle sowie Stecker des Lichtinstruments und somit bezüglich einer Lichtquelle koppeln lässt.

Wie bereits erwähnt, kann während den andauernden Manipulationen durch einen Operateur den das Lichtinstrument durch die Zugspannung aus dem Brennpunkt der Lichtquelle gezogen werden, was zu einer verminderten oder schlimmstenfalls sogar unterbrochenen Beleuchtung am chirurgischen Wirkungsort im Auge führt. Die magnetische Anziehungskraft zwischen dem Stecker des Lichtinstruments und der Buchse der Lichtquelle korrigiert dieses Verhalten, und die Einkoppelseite des Lichtinstruments liegt daher immer im Brennpunkt der Lichtquelle, was für eine maximale Lichtleistung / Beleuchtung sorgt, sowie eine stabile und reproduzierbare Lichtleistung des Beleuchtungssystems gewährleistet, während der Lichtleiter trotz hohen Zugspannungen unbeschädigt bleibt. Das Beleuchtungssystem sorgt somit für verbesserte und zuverlässige Beleuchtungsverhältnisse am chirurgischen Wirkungsort im Auge.

Zudem erlaubt der magnetisch anziehende bzw. anziehbare Stecker des Lichtinstruments eine einfache Positionierung zur Lichtquelle und abnehmbare Verbindung zur magnetisch anziehbaren bzw. anziehenden Buchse der Lichtquelle, da dazu keine Verriegelungsmechanismen wie z.B. Schraub- oder Bajonettverschlüsse betätigt werden müssen und aufgrund der magnetischen Anziehungskraft zwischen dem Stecker des Lichtinstruments und der Buchse der Lichtquelle nur ein geringer Kraftaufwand des Operateurs zu deren Verbindung resp. Kopplung mit der Lichtquelle erforderlich ist. Das Beleuchtungssystem verfügt somit über eine einfache Handhabung.

Die Buchse der Lichtquelle weist in einer ersten Variante vorzugsweise einen magnetisierbaren Materialanteil oder einen magnetisierten Materialanteil auf, wobei der magnetisierbare Materialanteil in der Buchse der Lichtquelle magnetisiert sein kann. Die Buchse der Lichtquelle weist in einer zweiten Variante einen magnetisch anziehbaren Materialanteil auf. In beiden Varianten ist der Materialanteil der Buchse der Lichtquelle derart gewählt, dass der Stecker des Lichtinstruments magnetisch zur Buchse der Lichtquelle angezogen wird. Das heisst, die Materialanteile des Steckers des Lichtinstruments, bzw. der Buchse der Lichtquelle werden so gewählt, dass zwischen den beiden Elementen eine magnetische Anziehungskraft bereitgestellt wird.

Somit ist es denkbar, dass der Stecker des Lichtinstruments einen magnetisch anziehbaren Materialanteil wie beispielsweise einen partikelförmigen, insbesondere eisenhaltigen Materialanteil, aufweist, und die Buchse der Lichtquelle einen magnetisch anziehenden Materialanteil, vorzugsweise einen magnetischen Materialanteil, aufweist. Eine solche Ausgestaltung ist bevorzugt, da der Stecker des Lichtinstruments dabei kostengünstig herstellbar ist und sich somit z.B. als Einwegprodukt für eine einmalige Verwendung eignet. Allerdings ist es ebenfalls denkbar, dass der Stecker des Lichtinstruments einen magnetisch anziehenden Materialanteil, vorzugsweise einen magnetischen Materialanteil, aufweist, und dass die Buchse der Lichtquelle einen magnetisch anziehbaren Materialanteil wie beispielsweise einen partikelförmigen, insbesondere eisenhaltigen Materialanteil, aufweist.

Im Kontext der unterschiedlichen Materialien bzw. Materialanteile sowie deren Materialeigenschaften wie beispielsweise Magnetisierung bzw. Entmagnetisierung der Buchse der Lichtquelle wird auf die obigen Erläuterungen im Kontext des Steckers des Lichtinstruments verwiesen. Diese Aussagen lassen sich analog für die Materialien bzw. Materialanteile und Eigenschaften der Buchse der Lichtquelle verstehen.

Also kann der magnetisierbare Materialanteil und/oder der magnetisierte Materialanteil des Beleuchtungssystems resp. der Buchse der Lichtquelle aus einem ferrimagnetischem und / oder ferromagnetischem Materialanteil bestehen.

Der magnetisierbare Materialanteil und/oder der magnetisierte Materialanteil und/oder der magnetisch anziehende Materialanteil ist in einer besonders bevorzugten Ausführungsform bevorzugt partikelförmig ausgebildet und wird in einer Kunststoffmatrix eingebettet. Der Stecker des Lichtinstruments ist also im Wesentlichen aus Kunststoff mit eingebetteten magnetisierbaren oder magnetisierten Partikeln hergestellt. Die Kunststoffmatrix wird vorzugsweise durch Spritzgiessen oder Extrudieren hergestellt, wobei die Partikel während des Spritzgiessens bzw. des Extrudierens einbettbar sind.

Wie bereits erwähnt erlaubt die Herstellung des Steckers des Lichtinstruments Spritzgussteil bzw. Extrusionsteil eine kostengünstige Herstellung in grossen Stückzahlen und ermöglicht deren Verwendung als Einwegteile, was insbesondere im Hinblick auf die Anforderungen betreffend Hygiene und Sterilität eines Operationssaales von grosser Bedeutung ist.

Die Buchse der Lichtquelle kann vollständig aus dem magnetisierbaren Material oder aus dem magnetisierten Material bestehen oder der magnetisierbare Materialanteil oder der magnetisierte Materialanteil kann nur bereichsweise, z.B. im Bereich des distalen Endes der Buchse der Lichtquelle, insbesondere in der weiteren Anschlagsfläche der Buchse der Lichtquelle, angeordnet sein.

Insbesondere ist es bevorzugt, dass der Stecker des Lichtinstruments einen magnetisierbaren Materialanteil oder einen magnetisierten Materialanteil in Partikelform aufweist, welche mindestens im Bereich seiner Anschlagsfläche oder vollständig im gesamten Stecker des Lichtinstruments verteilt ist, und dass die Buchse der Lichtquelle im Bereich ihrer weiteren Anschlagsfläche mindestens einen Magneten resp. mindestens eine Magnetspule aufweist.

Der magnetisierte Materialanteil kann als mindestens einen Magneten oder als mindestens eine Magnetspule ausgebildet sein. Insbesondere kann der mindestens eine Magnet oder die mindestens eine Magnetspule von einem Kunststoffmaterial hülsenförmig umspritzt sein.

Beim mindestens einen Magneten handelt es sich dabei bevorzugterweise um einen dauer- bzw. Permanentmagneten.

Die Buchse der Lichtquelle weist vorzugsweise einen im Wesentlichen zylinderförmigen Körper auf, welcher im Inneren einen Hohlraum definiert. Der Hohlraum kann in eine Eintrittsöffnung am proximalen Ende und in eine Austrittsöffnung am distalen Ende der Buchse der Lichtquelle münden, wobei sich der Lichtleiter durch die Austrittsöffnung via den Hohlraum zumindest teilweise durch den Hohlraum erstrecken kann.

Es ist bevorzugt, dass der Lichtleiter dabei im Inneren der Buchse der Lichtquelle, insbesondere im Bereich der Eintritts- und Austrittsöffnung der Buchse der Lichtquelle, fest gelagert ist.

Es ist bevorzugt, dass die Buchse der Lichtquelle im Bereich ihres Hohlraums wie auch ihr Hohlraum jeweils einen konstanten Durchmesser aufweisen.

Es ist aber auch denkbar, dass die Buchse der Lichtquelle und/oder ihr Hohlraum entgegengesetzt zueinander oder gleichgesetzt zueinander konisch zulaufend oder konisch aufweitend ausgebildet sind.

Am proximalen Ende der Buchse der Lichtquelle kann ein Justierflansch angeordnet sein, dessen Durchmesser grösser als der Durchmesser des im Wesentlichen zylinderförmigen Körpers der Buchse der Lichtquelle ist.

Der Justierflansch ist über eine Schraubverbindung mit der die Steckerbuchse am Lichtinstrument fest fixiert ist. Über ein Feingewinde zwischen dem Buchsenkörper und dem Justierflansch kann der Abstand zur Fokuslinse derart justiert werden, dass das proximale Ende des Lichtleiters im Brennpunkt liegt. Nach Justage kann der Justierflansch fixiert werden und die Steckerbuchse, die mit dem Justierflansch ein Bestandteil der Steckerbuchse ist, wird so ein fester unverrückbarer Bestandteil der Lichtquelle

Die Buchse der Lichtquelle und der Justierflansch können dabei als zwei separate Teile ausgebildet sein.

Der Justierflansch ist im Querschnitt vorzugsweise im Wesentlichen T-förmig ausgebildet und erstreckt sich durch die Eintrittsöffnung der Buchse der Lichtquelle zumindest teilweise in den Hohlraum der Buchse der Lichtquelle hinein.

Eine durchgehende Öffnung kann sich mittig durch den Justierflansch hindurch erstrecken, durch welche Öffnung der Lichtleiter in den Hohlraum der Buchse der Lichtquelle einführbar ist.

Wie oben beschrieben ist es bevorzugt, dass der Lichtleiter in einem Führungsrohr gelagert ist, welches sich durch den Stecker hindurch erstreckt und welches fest mit dem Stecker in Verbindung steht. Vorzugsweise erstreckt sich das Führungsrohr mit seinem proximalen Ende aus dem Stecker des Lichtinstrumentes hinaus, so dass das Führungsrohr in eine Buchse einschiebbar ist. Besonders bevorzugt erstreckt sich das proximale Ende des Führungsrohrs über die gleiche Distanz aus dem Stecker wie der Lichtleiter selbst. Das heisst, der Lichtleiter ist von der Anschlagsfläche des Steckers gesehen im Wesentlichen über seine gesamte Länge von der Anschlagsfläche gesehen durch das Führungsrohr umgeben. Das proximale Ende des Führungsrohrs kommt im Wesentlichen in den Brennpunkt zu liegen.

Der verbundene Zustand bezeichnet dabei die abnehmbare Verbindung des Lichtinstruments, also des Steckers des Lichtinstruments mit Lichtleiter bzw. zusätzlich mit dem Führungsrohr, mit der Buchse der Lichtquelle. Die abnehmbare Verbindung erfolgt dabei aufgrund der magnetischen Anziehungskraft zwischen dem Stecker des Lichtinstruments und der Buchse der Lichtquelle. Ein unverbundener Zustand liegt vor, wenn das Lichtinstrument und die Buchse der Lichtquelle ausserhalb der Reichweite ihrer magnetischen Anziehungskraft liegen.

Die magnetische Anziehungskraft kann auch als magnetische Zugkraft bezeichnet werden und der Kraft entsprechen, welche vom magnetisch anziehenden Materialanteil auf den magnetisch anziehbaren Materialanteil ausgeübt wird.

Eine magnetische Zugkraft zwischen dem Stecker des Lichtinstruments und der Buchse der Lichtquelle beträgt bevorzugterweise zwischen 0.1 N und 5 N, vorzugsweise zwischen 0.5 N und 2 N, oder die magnetische Zugkraft beträgt bevorzugter Weise gleichviel oder mehr als 2 N.

Der Stecker des Lichtinstruments und die Buchse der Lichtquelle können zwischen einen Abstand von 1 mm und 30 mm, vorzugsweise zwischen 5 mm und 20 mm magnetisch anziehbar ausgebildet sein, oder der Stecker des Lichtinstruments und die Buchse der Lichtquelle können über einen Abstand von mindestens 10 mm, vorzugsweise von mindestens 20 mm magnetisch anziehbar ausgebildet sein.

Vorzugsweise erleichtert die Zugkraft über einen bestimmten Abstand das Stecken des Steckers. Folglich muss der Stecker dann nicht von Hand auf Anschlag geschoben werden, sondern der Stecker wird, sobald die Magnetkraft wirkt, automatisch auf Anschlag gezogen.

Es ist bevorzugt, dass der Stecker des Lichtinstruments als Handgriff ausgebildet ist.

Dies erleichtert und verbessert die Handhabung des Beleuchtungssystems, da der Operateur den Stecker des Lichtinstruments in Gestalt eines Handgriffs auch mit übergezogenen Operationshandschuhen fassen und anschliessend mit geringem Kraftaufwand mit der Buchse der Lichtquelle des Beleuchtungsgerätes verbinden kann. Der Stecker des Lichtinstruments wird, bei kleinem Abstand, automatisch auf den Anschlag der Buchse der Lichtquelle gezogen.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1a: eine perspektivische Ansicht eines ophthalmischen Beleuchtungssystems mit einem Lichtinstrument und einer Lichtquelle mit einer Buchse;
- Fig. 1b: eine perspektivische Ansicht der Steckverbindung eines ophthalmisches Beleuchtungssystems mit einem opthalmischen Lichtinstrument und der Buchse der Lichtquelle in unverbundenem Zustand, wobei sich ein Lichtleiter durch einen Stecker des Lichtinstruments erstreckt nach den Figuren 1a;
- Fig. 2: eine perspektivische Ansicht der Steckverbindung des opthalmischen Beleuchtungssystems gemäss Figur 1, wobei sich der Lichtleiter zusätzlich in die Buchse der Lichtquelle erstreckt;
- Fig. 3: eine perspektivische Ansicht der Steckverbindung eines ophthalmischen Beleuchtungssystems mit dem opthalmischen Lichtinstrument und der Buchse der Lichtquelle in verbundenem Zustand;
- Fig. 4: eine Schnittansicht durch die Steckverbindung des ophthalmischen Beleuchtungssystems in unverbundenem Zustand gemäss Figur 2;
- Fig. 5: eine Schnittansicht durch die Steckverbindung des ophthalmischen Beleuchtungssystems im verbundenen Zustand gemäss Figur 3;
- Fig. 6: eine Detailansicht des Bereiches A gemäss Figur 5;
- Fig. 7: eine perspektivische Ansicht des Steckers des Lichtinstruments, wobei sich der Lichtleiter durch den Stecker des Lichtinstruments hindurch erstreckt;
- Fig. 8: eine Schnittansicht durch den Stecker des Lichtinstruments mit Lichtleiter gemäss Figur 7;
- Fig. 9: eine Schnittansicht durch das proximale Ende des Stecker des Lichtinstruments; und
- Fig. 10: eine Schnittansicht durch das distale Ende des Steckers des Lichtinstruments.

### BESCHREIBUNG BEVORZUGTER AUSFÜHRUNGSFORMEN

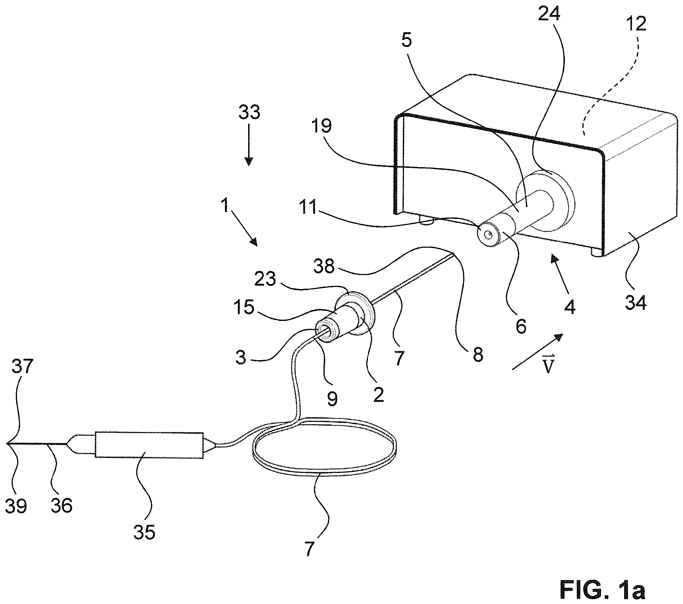

Die Figur 1a zeigt ein ophthalmisches Beleuchtungssystems. Das opthalmische Beleuchtungssystem umfasst ein Lichtinstrument 33 mit einem Stecker 1 und eine Lichtquelle 12 mit einer Buchse 4. Die Lichtquelle 12 ist hier in einem Gehäuse 34 angeordnet und die Buchse der Lichtquelle 4 ist am Gehäuse 34 fest befestigt.

Das Lichtinstrument 33 ist in der gezeigten Ausführung ein Beleuchtungselement, welches ausschliesslich für die Beleuchtung vorgesehen ist. Alternativ kann das Lichtinstrument aber auch einen integralen Teil eines beleuchtetetn Instrumentes sein, wie beispielsweise eines beleuchteten Cutters, einer beleuchteten Lasersonde, einer beleuchteten Pinzette, etc.

Das Lichtinstrument 33 ist hier beabstandet von der Buchse der Lichtquelle 4 und vom Gehäuse 34 dargestellt. Das Lichtinstrument 33 kann, wie in der Folge beschrieben wird, über den Stecker des Lichtinstruments 1 mit der Buchse der Lichtquelle 4 verbunden werden.

Das Lichtinstrument 33 umfasst in der gezeigten Ausführungsform im Wesentlichen einen Handgriff 35, einen Lichtleiter 7 und einen Stecker des Lichtinstruments 1. Der Lichtleiter 7 steht, wie unten beschrieben, mit dem Stecker des Lichtinstruments 1 in fester Verbindung und über die Lichtquelle 12 wird Licht in den Lichtleiter eingekoppelt. Weiter steht der Lichtleiter 7 mit dem Handgriff 35 und der Kanüle 36 fest in Verbindung. Der Handgriff 35 umfasst zudem eine Kanüle 36, über welche das Licht dann ausgekoppelt werden kann. Durch die Kanüle 36 erstreckt sich wiederum der Lichtleiter 7 bis hin zum distalen Ende 39 der Kanüle 36. Das distale Ende 37 des Lichtleiters 7 befindet sich am distalen Ende 39 der Kanüle 36.

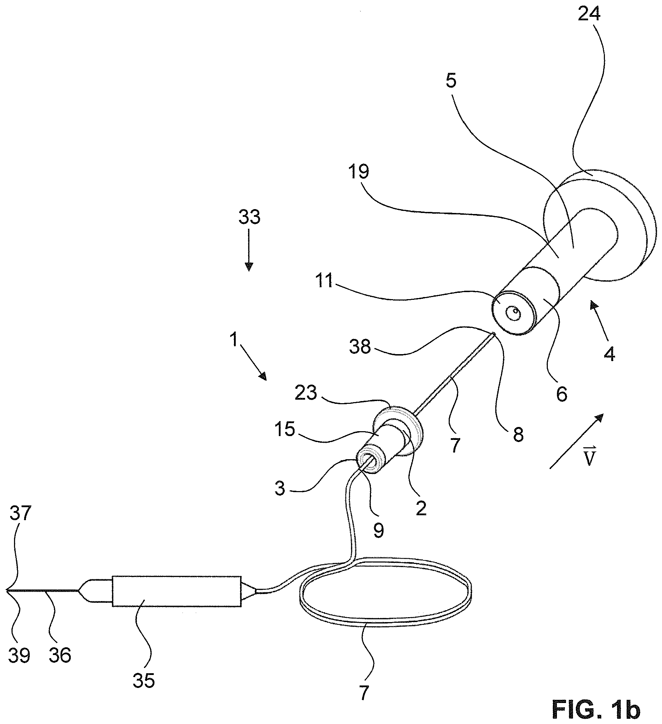

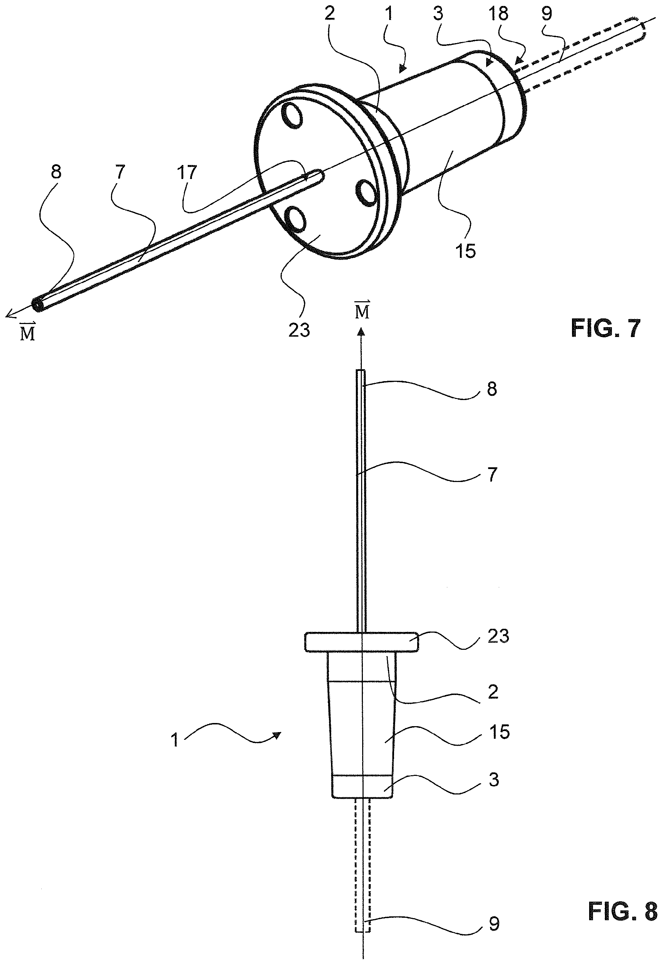

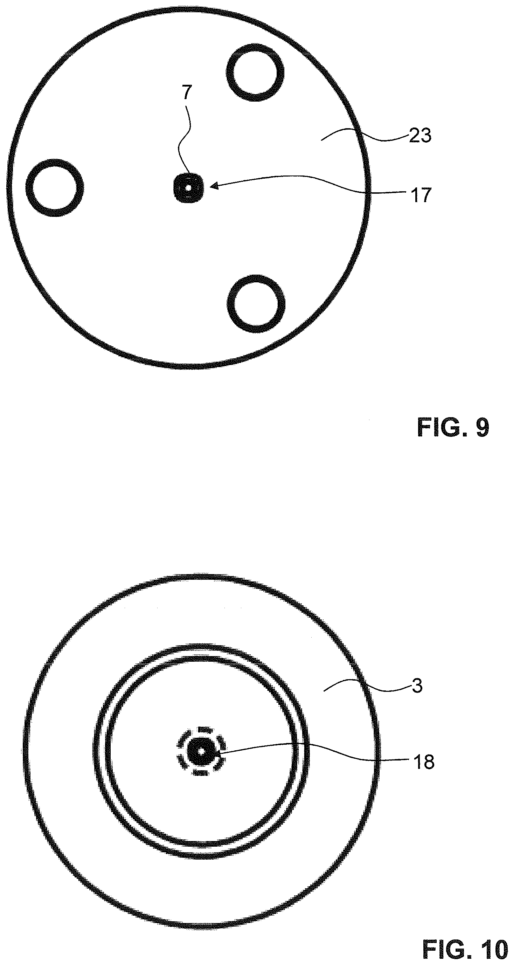

In den Figuren 1b bis 5 ist jeweils ein ophthalmisches Beleuchtungssystem mit einem Stecker des Lichtinstruments und einer Buchse der Lichtquelle gezeigt, während in den Figuren 7 bis 10 der Stecker des Lichtinstrumentes alleine dargestellt ist.

Das ophthalmische Beleuchtungssystem umfasst dabei ein ophthalmisches Lichtinstrument sowie eine Lichtquelle mit Buchse 4. Das Lichtinstrument wiederum umfasst einen Stecker des Lichtinstruments 1 mit einer Anschlagsfläche 23 sowie einen am Stecker des Lichtinstruments 1 gelagerten Lichtleiter 7 zum Zuleiten von Licht an eine chirurgische Stelle im Auge. Der Lichtleiter 7 weist ein proximales Ende 8 zum Einkoppeln von Licht 10 von einer Lichtquelle 12 sowie ein distales Ende 37 zum Emittieren des eingekoppelten Lichts 10 auf. Das distale Ende 37 des Lichtleiters 7 befindet sich hier am distalen Ende 39 der Kanüle 36. Die Kanüle 36 kann während eines Operationsvorgangs an die vom Operationspersonal gewünschte Stelle bewegt werden. Der Stecker des Lichtinstruments 1 ist magnetisch anziehbar bzw. anziehend ausgebildet und die Buchse der Lichtquelle 4 ist ebenfalls magnetisch anziehbar bzw. anziehend ausgebildet und weist eine weitere Anschlagsfläche 11 auf, so dass die Buchse der Lichtquelle 4 über ihre weitere Anschlagsfläche 11 derart zur Lichtquelle positionierbar und mit der Anschlagsfläche 23 des Steckers des Lichtinstruments 1 abnehmbar verbindbar ist, dass das proximale Ende 8 des Lichtleiters 7 im Brennpunkt 14 der Lichtquelle 12 zu liegen kommt, so dass eine Einkopplung des Lichts 10 in das Lichtinstrument im Brennpunkt 14 der Lichtquelle 12 erfolgt.

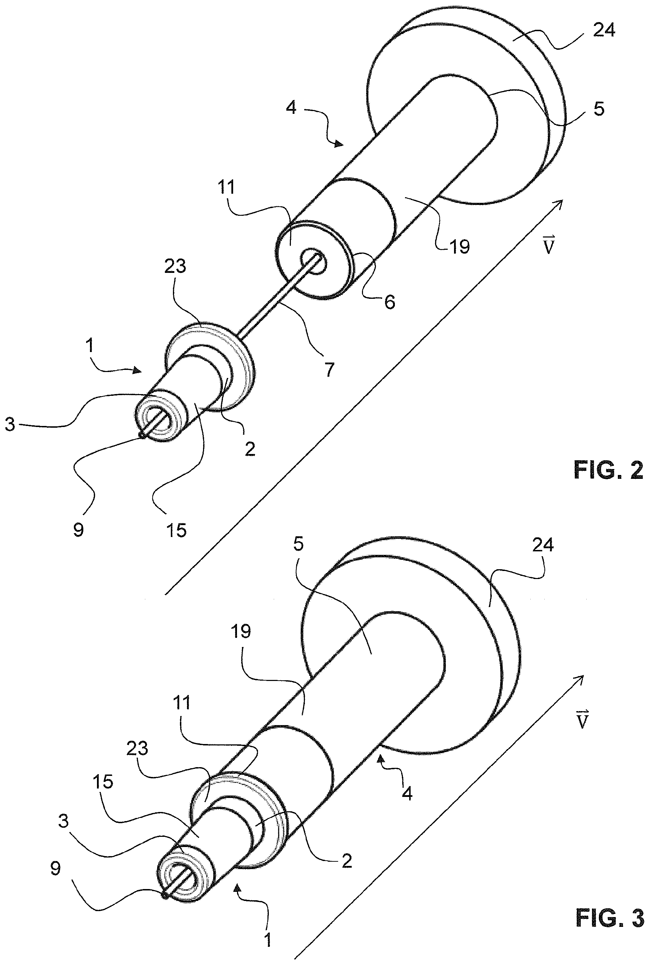

Das heisst, die Buchse der Lichtquelle 4 kann separat vom Lichtinstrument in einem unverbundenen Zustand gemäss den Figuren 1b und 2 sowie in einem verbundenen Zustand gemäss der Figur 3 vorliegen.

Im unverbundenen Zustand gemäss Figur 1 erstreckt sich der Lichtleiter 7 vollständig durch den Stecker des Lichtinstruments 1 hindurch und ragt mit seinem distalen Ende 37 über das distale Ende 3 des Steckers des Lichtinstruments 1 und mit seinem proximalen Ende 8 über das proximale Ende 2 des Steckers des Lichtinstruments 1 hinaus. Um das Lichtinstrument, hier also den Stecker des Lichtinstruments 1 mit dem Lichtleiter 7, mit der Buchse der Lichtquelle 4 zum Beleuchtungssystem zu verbinden, wird zunächst das proximale Ende 8 des Lichtleiters 7 in das distale Ende 6 der Buchse der Lichtquelle 4 eingeführt. Diese Situation entspricht dem immer noch unverbundenen Beleuchtungssystem gemäss Figur 2. Nachdem der Lichtleiter 7 nicht nur im Stecker des Lichtinstruments 1 sondern zumindest teilweise auch in der Buchse der Lichtquelle 4 aufgenommen ist, und sich die Buchse der Lichtquelle 4 sowie der Stecker des Lichtinstruments 1 innerhalb einem bestimmten Abstand d zueinander befinden, so führt eine magnetische Anziehung zwischen dem magnetisch anziehbaren Stecker des Lichtinstruments 1 und der magnetisch anziehenden Buchse der Lichtquelle 4, bzw. zwischen dem magnetisch anziehenden Stecker des Lichtinstruments 1 und der magnetisch anziehbaren Buchse der Lichtquelle 4 zu einer Verbindung des Steckers des Lichtinstruments 1 mit der Buchse der Lichtquelle 4 entlang einer Verbindungsrichtung V. Diese Situation entspricht dem verbundenen Beleuchtungssystem gemäss Figur 3. In diesem verbundenen Zustand liegen das proximale Ende 2 des Steckers des Lichtinstruments 1 und das distale Ende 6 der Buchse der Lichtquelle 4 flächig aufeinander auf.

Um das Beleuchtungssystem zu trennen, also den Stecker des Lichtinstruments 1 von der Buchse der Lichtquelle 4 zu entfernen, zieht das Operationspersonal entgegen der Verbindungsrichtung V den Stecker des Lichtinstruments 1 so weit von der Buchse der Lichtquelle 4 weg, bis sich die Anschlagsfläche 23 des Steckers des Lichtinstruments 1 und die weitere Anschlagsfläche 11 der Buchse der Lichtquelle 4 ausserhalb der Reichweite der magnetischen Anziehungskraft befindet. Das Beleuchtungssystem liegt dann wieder in seinem unverbundenen Zustand vor und bei Bedarf kann der Stecker eines neuen Lichtinstruments mit der Buchse der Lichtquelle zu einem neuen Beleuchtungssystem verbunden werden.

Wie aus den Schnittansichten 4 resp. 5 hervorgeht, weist der Stecker des Lichtinstruments 1 einen im Wesentlichen zylinderförmigen Körper 15 auf, welcher im Inneren einen Hohlraum 16 definiert. Der Hohlraum 16 mündet dabei in eine Eintrittsöffnung 17 am proximalen Ende 2 sowie in eine Austrittsöffnung 18 am distalen Ende 3 des Steckers des Lichtinstruments 1. Die Anschlagsfläche 23 am proximalen Ende 2 des Steckers des Lichtinstruments 1 ist als einen Flansch ausgebildet, dessen Durchmesser DF grösser als der Durchmesser DS des im Wesentlichen zylinderförmigen Körpers 15 des Steckers des Lichtinstruments 1 ist. In diesen Figuren sind der im Wesentlichen zylinderförmige Körper 15 des Steckers des Lichtinstruments 1 und die als Flansch ausgebildete Anschlagsfläche 23 einstückig ausgebildet.

Der Hohlraum 16 weist hier zwei Abschnitte unterschiedlichen Durchmessers auf. Der erste Abschnitt mündet in die Eintrittsöffnung 17 am proximalen Ende 2 des Steckers des Lichtinstruments 1 und weist einen Durchmesser auf, der im Wesentlichen dem Aussendurchmesser des Lichtleiters 7 entspricht. Der zweite Abschnitt schliesst sich in distaler Richtung an diesen ersten Abschnitt an und mündet in die Austrittsöffnung 18 am distalen Ende 3 des Steckers des Lichtinstruments 1, wobei der zweite Abschnitt einen Durchmesser aufweist, der grösser ist als der Durchmesser im ersten Abschnitt.

Dieser Bereich des Steckers des Lichtinstruments, also dort, wo der Hohlraum 16 im Stecker des Lichtinstruments ausgebildet ist, ist entlang der distalen Richtung gesehen, also ausgehend von der Eintrittsöffnung 17 in Richtung der Austrittsöffnung 18 des Steckers des Lichtinstruments, konisch zulaufend ausgebildet, während der darin ausgebildete Hohlraum 16 in diesem Bereich entlang dieser distalen Richtung konisch aufweitend ausgebildet ist.

Die Buchse der Lichtquelle 4 weist ebenfalls einen im Wesentlichen zylinderförmigen Körper 19 auf, welcher im Inneren einen Hohlraum 20 definiert, und wobei der Hohlraum 20 in eine Eintrittsöffnung 21 am proximalen Ende 5 und in eine Austrittsöffnung 22 am distalen Ende 6 der Buchse der Lichtquelle 4 mündet. Am proximalen Ende 5 der Buchse der Lichtquelle 4 ist ein Justierflansch 24 angeordnet, dessen Durchmesser DJ grösser als der Durchmesser DB des im Wesentlichen zylinderförmigen Körpers 19 der Buchse der Lichtquelle 4 ist. Wie in diesen Figuren ersichtlich ist, ist der Justierflansch 24 im Querschnitt im Wesentlichen T-förmig ausgebildet und erstreckt sich durch die Eintrittsöffnung 21 der Buchse der Lichtquelle 4 zumindest teilweise in den Hohlraum 20 der Buchse der Lichtquelle 4 hinein. Weiter weist der Justierflansch eine durchgehende Öffnung 25 auf, welche sich mittig durch den Justierflansch 24 hindurch erstreckt, und durch deren Öffnung 25 der Lichtleiter 7 in den Hohlraum 20 der Buchse der Lichtquelle 4 einführbar ist. Während der Stecker des Lichtinstruments 1 über einen konisch zulaufenden Steckerkörper des Lichtinstruments verfügt und dessen Hohlraum konisch aufweitend ist, weist die Buchse der Lichtquelle 4 im Bereich ihres Hohlraums 20 einen konstanten Durchmesser auf.

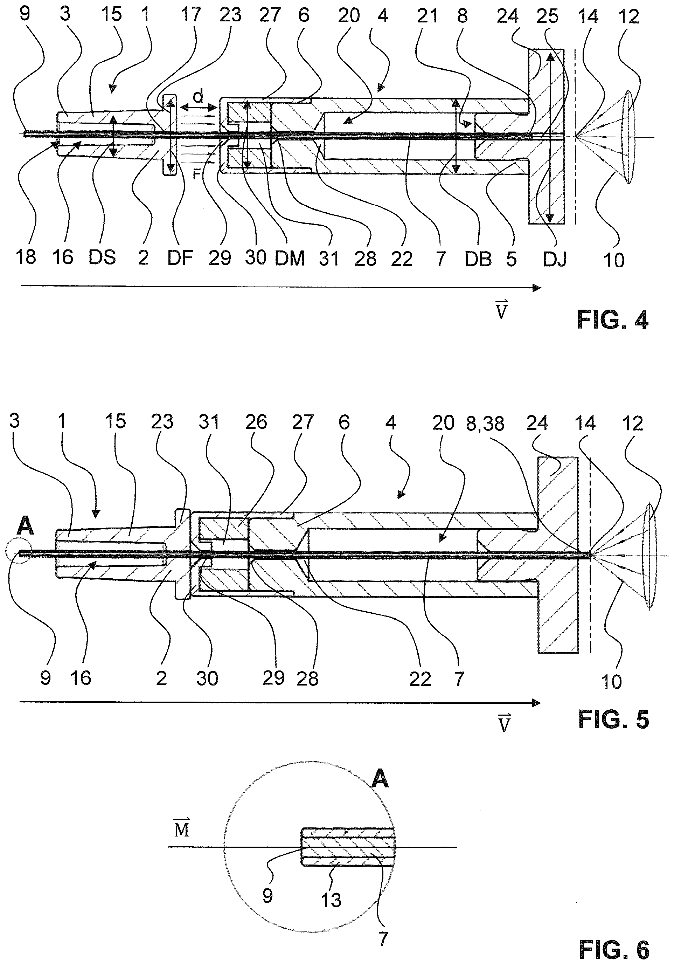

Der Justierflansch 24 ist hier über eine Schraubverbindung mit der Buchse 4 in Verbindung. Über die Schraubverbindung, welche vorzugsweise ein Feingewinde ist, kann der Abstand zur Fokuslinse der Lichtquelle 12 derart justiert werden, dass das proximale Ende 8 des Lichtleiters 7 in der Fokusebene liegt. Nach Justage wird der Justierflansch 24 fixiert und die Buchse 4 liegt dann in fester und unverrückbarer Lager zur Lichtquelle. Im noch immer unverbundenen Zustand gemäss Figur 4 erstreckt sich der Lichtleiter 7 mit seinem distalen Ende 37 durch die Eintrittsöffnung 17 via den Hohlraum 16 durch die Austrittsöffnung 18 des Steckers des Lichtinstruments 1 vollständig durch den Stecker des Lichtinstruments 1 hindurch. Mit seinem proximalen Ende 8 erstreckt sich der Lichtleiter 7 durch die Austrittsöffnung 22 via den Hohlraum 20 zumindest teilweise durch die Eintrittsöffnung 21 der Buchse der Lichtquelle 4 durch. Insbesondere wird der proximale Bereich 8 des Lichtleiters 7 dabei zumindest teilweise in die Öffnung 25 des Justierflansches 24 eingeführt, ragt aber noch nicht über das proximale Ende des Justierflansches 24 resp. das proximale Ende 5 der Buchse 4 hinaus.

Sobald sich der Stecker des Lichtinstruments 1 und die Buchse der Lichtquelle 4 innerhalb eines bestimmten Abstandes d zueinander befinden, führt die magnetische Anziehungskraft F zwischen dem magnetisch anziehenden bzw. anziehbaren Stecker des Lichtinstruments 1 resp. Buchse der Lichtquelle 4 zu einer vollständigen Verbindung des Steckers des Lichtinstruments 1 mit der Buchse der Lichtquelle 4, resp. zwischen deren Anschlagsflächen 23, 11. Die magnetische Anziehung zwischen dem Stecker des Lichtinstruments 1 und der Buchse der Lichtquelle 4 entspricht dabei einer magnetischen Zugkraft zwischen ca 0.5 N und 2 N, wodurch der Stecker des Lichtinstruments 1 und die Buchse der Lichtquelle 4 in einen Abstand d zwischen ca. 5 mm und 20 mm magnetisch anziehbar sind.

Während unterschiedliche Ausgestaltungen des magnetisch anziehbar bzw. anziehend ausgebildeten Steckers des Lichtinstruments 1 und der magnetisch anziehbar bzw. anziehend ausgebildeten Buchse der Lichtquelle 4 im Rahmen der gegenwärtigen Erfindung möglich sind, weist der in den Figuren gezeigte Stecker des Lichtinstruments 1 partikelförmiges magnetisierbares Material oder magnetisiertes Material auf, welches durch Spritzgiessen oder Extrudieren in eine Kunststoffmatrix hergestellt wurde. Wird während dem Spritzgiessen oder Extrudieren ein starkes Magnetfeld angelegt, so wird das magnetisierbare Material magnetisiert, wird kein Magnetfeld angelegt, so wird das magnetisierbare Material nicht magnetisiert. Der Stecker des Lichtinstruments 1 ist hier also vollständig aus Kunststoff hergestellt, in welchem magnetisierbare resp. magnetisierte Partikel eingeschlossen sind. Die Buchse der Lichtquelle 4 weist hier im Bereich ihres distalen Endes 6 das magnetisierte Material in Form eines Dauermagneten 26 auf, welche von einer Hülse aus Metall oder Kunststoff umgeben ist. Der Dauermagnet 26 ist hier ringförmig ausgebildet und umringt den Lichtleiter 7 vollständig. Der Durchmesser DM des Dauermagneten sowie der Durchmesser DB der Buchse der Lichtquelle 4 im Bereich ihres distalen Endes 6, wo der Dauermagnet 26 zu liegen kommt, ist etwas kleiner als der Durchmesser DB der Buchse der Lichtquelle 4 im Bereich ihres Hohlraums 20, so dass die Hülse 27 wie ein Deckel über den Dauermagneten 26 zu liegen kommt und sich dabei übergangslos an das distale Ende 6 der Buchse der Lichtquelle 4 anschliesst. Mit anderen Worten hat die Hülse 27 somit denselben Durchmesser wie die Buchse der Lichtquelle 4 im Bereich ihres Hohlraums 20 und die Hülse 27 bildet somit mit ihrer Stirnseite die weitere Anschlagsfläche 30 für die Anschlagsfläche 23 des Steckers des Lichtinstruments 1. Die Hülse 27 weist ebenfalls einen Hohlraum 31 auf, welcher in proximaler Richtung in eine Eintrittsöffnung 28 und in distaler Richtung in eine Austrittsöffnung 29 mündet.

Wenn also das Beleuchtungssystem über eine Buchse der Lichtquelle 4 mit aufgespritzer Hülse 27 verfügt, so erstreckt sich der aufgenommene Lichtleiter 7 im verbundenen Zustand entlang der Verbindungsrichtung V, was der proximalen Richtung entspricht, von der Austrittsöffnung 18 des Steckers des Lichtinstruments 1 via dessen Hohlraum 16 durch dessen Eintrittsöffnung 17 in die Austrittsöffnung 29 der Hülse 27, weiter via deren Hohlraum 31 durch deren Eintrittsöffnung 28 in die Austrittsöffnung 22 der Buchse der Lichtquelle 4, und weiter via deren Hohlraum 20 durch die Öffnung 25 im Justierflansch 24 sowie durch die Eintrittsöffnung 21 der Buchse der Lichtquelle 4 proximal aus der Buchse der Lichtquelle 4 heraus.

Wie aus Figur 5 hervorgeht, liegen dann die Anschlagsfläche 23 des Steckers des Lichtinstruments 1 und die weitere Anschlagsfläche 30 der Hülse 27 flächig aufeinander auf und das proximale Ende 8 des Lichtleiters 7 liegt im Brennpunkt 14 der Lichtquelle 12, so dass eine Einkopplung des Lichts 10 in das Lichtinstrument im Brennpunkt 14 der Lichtquelle 12 erfolgt. Hier handelt es sich um Licht 10 aus einer Lichtquelle 12, welche das emittierte Licht 10 mittels einer Linse in den Brennpunkt 14 fokussiert.

Vorzugsweise steht der Lichtleiter 7 über ein Führungsrohr 13 mit dem Stecker fest in Verbindung. Das Führungsrohr 13 erstreckt sich in der gezeigten Ausführungsform vom proximalen Ende 8 des Lichtleiters 7 durch den Stecker 1 des Lichtinstruments hindurch. Das Führungsrohr 13 umfasst dabei ein distales Ende 9, aus welchen dann der Lichtleiter, so wie in den Figuren 1a und 1b gezeigt, hinaus ragt. Das proximale Ende 8 des Lichtleiters 7 liegt hier im Bereich des proximalen Endes 38 des Führungsrohrs 13.

Figur 6 zeigt eine Detailansicht des Bereiches A gemäss der Figur 5, woraus ersichtlich wird, dass das Führungsrohr 13 zur Aufnahme und zur Führung des Lichtleiters 7 betriebstechnisch mit dem Stecker des Lichtinstruments 1 und der Buchse der Lichtquelle 4 gekoppelt werden kann. Dieses Führungsrohr 13 umgibt den Lichtleiter 7 hülsenförmig und erstreckt sich somit wie der Lichtleiter 7 durch die Austrittsöffnung 18 des Steckers des Lichtinstruments 1 in dessen Hohlraum 16 durch dessen Eintrittsöffnung 17 weiter über die Austrittsöffnung 22 der Buchse der Lichtquelle 4 durch deren Hohlraum 20 und, im verbundenen Zustand des Beleuchtungssystems, durch deren Eintrittsöffnung 21 proximal aus der Buchse der Lichtquelle 4 heraus.

Wie insbesondere aus den Figuren 7 bis 10 hervorgeht, kann das Führungsrohr 13 als Führung für den Lichtleiter 7 angesehen werden, welche im Wesentlichem um eine Mittelachse M rotationssymmetrisch ausgestaltet ist und sich mittig durch den Stecker des Lichtinstruments 1 und die Buchse der Lichtquelle 4 hindurch erstreckt. Aus dem distalen Ende des Führungsrohrs 13 ragt der darin aufgenommene Lichtleiter 7 hinaus und wird durch den Handgriff 35 zum distalen Ende 39 der Kanüle 36 geführt. Diese Kanüle 36 kann mit dem innenliegenden Lichtleiter 7 zu Beleuchtungszwecken in das Auge eingeführt werden.

### BEZUGSZEICHENLISTE

- 1: Stecker des Lichtinstruments
- 2: proximales Ende Stecker
- 3: distales Ende Stecker
- 4: Buchse der Lichtquelle
- 5: proximales Ende Buchse
- 6: distales Ende Buchse
- 7: Lichtleiter
- 8: proximales Ende Lichtleiter
- 9: distales Ende Führungsrohr
- 10: Licht
- 11: Anschlagsfläche Buchse
- 12: Lichtquelle
- 13: Führungsrohr
- 14: Brennpunkt
- 15: Steckerkörper
- 16: Hohlraum Stecker
- 17: Eintrittsöffnung Stecker
- 18: Austrittsöffnung Stecker
- 19: Buchsenkörper
- 20: Hohlraum Buchse
- 21: Eintrittsöffnung Buchse
- 22: Austrittsöffnung Buchse
- 23: Anschlagsfläche Stecker
- 24: Justierflansch
- 25: Öffnung
- 26: Magnet
- 27: Hülse
- 28: Eintrittsöffnung Hülse
- 29: Austrittsöffnung Hülse
- 30: Anschlagsfläche Buchse
- 31: Hohlraum Hülse
- 32: ophthalmisches Beleuchtungssystem
- 33: Lichtinstrumente
- 34: Gehäuse
- 35: Handgriff
- 36: Kanüle
- 37: distales Ende Lichtleiter
- 38: proximales Ende Führungsrohr
- 39: distales Ende der Kanüle
- DB: Durchmesser Buchse
- DF: Durchmesser Flansch
- DJ: Durchmesser Justierflansch
- DM: Durchmesser Magnet
- DS: Durchmesser Stecker
- V: Verbindungsrichtung
- M: Mittelachse

## Patentansprüche

1. Ophthalmisches Lichtinstrument (33) umfassend
einen Stecker des Lichtinstruments (1) mit einer Anschlagsfläche (23); und
einen am Stecker des Lichtinstruments (1) gelagerten Lichtleiter (7) zum Zuleiten von Licht (10) an eine chirurgische Stelle im Auge, wobei der Lichtleiter (7) ein proximales Ende (8) zum Einkoppeln von Licht (10) von einer Lichtquelle (12) sowie ein distales Ende (37) zum Emittieren von Licht (10) aufweist,
wobei der Stecker des Lichtinstruments (1) magnetisch anziehbar bzw. anziehend ausgebildet ist und über seine Anschlagsfläche (23) zur Lichtquelle positionierbar und zur Lichtquelle abnehmbar verbindbar ist,
wobei der Stecker des Lichtinstruments (1) einen magnetisierbaren Materialanteil oder einen magnetisierten Materialanteil aufweist,
wobei der magnetisierbare Materialanteil im Stecker des Lichtinstruments (1) magnetisiert ist, und
wobei der magnetisierbare Materialanteil und/oder der magnetisierte Materialanteil aus einem ferrimagnetischen Materialanteil besteht, und/oder
wobei der magnetisierbare Materialanteil und/oder der magnetisierte Materialanteil aus einem ferromagnetischen Materialanteil besteht,
**dadurch gekennzeichnet, dass** der magnetisierbare Materialanteil und/oder der magnetisierte Materialanteil partikelförmig ist und in eine Kunststoffmatrix eingebettet ist.

2. Lichtinstrument gemäss Anspruch 1, wobei die Kunststoffmatrix durch Spritzgiessen oder Extrudieren herstellbar ist, wobei die Partikel während des Spritzgiessens bzw. des Extrudierens einbettbar sind, und wobei der magnetisierbare Materialanteil insbesondere während des Spritzgiessens oder während des Extrudierens oder nach dem Spritzgiessen bzw. durch Anlegen eines Magnetfeldes magnetisiert wird.

3. Lichtinstrument gemäss einem der vorhergehenden Ansprüche, wobei der Stecker des Lichtinstruments (1) vollständig aus dem magnetisierbaren Materialanteil oder aus dem magnetisierten Materialanteil besteht, oder
wobei der magnetisierbare Materialanteil oder der magnetisierte Materialanteil im Bereich des proximalen Endes (2) des Steckers des Lichtinstruments (1), insbesondere in der Anschlagsfläche (23) des Steckers des Lichtinstruments (1), angeordnet ist.

4. Lichtinstrument gemäss einem der vorhergehenden Ansprüche, wobei der Stecker des Lichtinstruments (1) einen im Wesentlichen zylinderförmigen Körper (15) aufweist welcher im Inneren einen Hohlraum (16) definiert,
wobei der Hohlraum (16) in eine Eintrittsöffnung (17) am proximalen Ende (2) und in eine Austrittsöffnung (18) am distalen Ende (3) des Steckers des Lichtinstruments (1) mündet, und
wobei sich der Lichtleiter (7) durch die Eintrittsöffnung (17) via den Hohlraum (16) durch die Austrittsöffnung (18) hindurch erstreckt.

5. Lichtinstrument gemäss Anspruch 4, wobei die Anschlagsfläche (23) am proximalen Ende (2) des Steckers des Lichtinstruments (1) als einen Flansch ausgebildet ist, dessen Durchmesser (DF) grösser als der oder gleich dem Durchmesser (DS) des im Wesentlichen zylinderförmigen Körpers (15) des Steckers des Lichtinstruments (1) ist.

6. Lichtinstrument gemäss einem der vorhergehenden Ansprüche, wobei der Lichtleiter (7) am bzw. im Stecker (1) fest gelagert ist, wobei der Lichtleiter (7) insbesondere über ein Führungsrohr (13), welches sich durch den Stecker (1) hindurch erstreckt, im Stecker (1) fest gelagert ist.

7. Ophthalmisches Beleuchtungssystem (32) umfassend ein Lichtinstrument gemäss einem der vorhergehenden Ansprüche 1 bis 6 und eine Buchse der Lichtquelle (4) mit einer weiteren Anschlagsfläche (11), **dadurch gekennzeichnet,**
**dass** die Buchse der Lichtquelle (4) magnetisch anziehbar bzw. anziehend ausgebildet ist und dass die Anschlagsfläche des Steckers (23) mit der weiteren Anschlagsfläche der Buchse (11) in Kontakt kommt, so dass der Lichtleiter (7) derart zur Lichtquelle positionierbar und mit dem Stecker des Lichtinstruments (1) abnehmbar verbindbar ist, dass das proximale Ende (8) des Lichtleiters (7) im Brennpunkt der Lichtquelle zu liegen kommt, so dass eine Einkopplung von Licht (10) im Brennpunkt (14) der Lichtquelle (10) erfolgt.

8. Beleuchtungssystem gemäss Anspruch 7, wobei die Buchse der Lichtquelle (4) einen magnetisierbaren Materialanteil oder einen magnetisierten Materialanteil oder einen magnetisch anziehbaren Materialanteil aufweist, und wobei der Materialanteil der Buchse der Lichtquelle derart gewählt ist, dass der Stecker des Lichtinstruments magnetisch zur Buchse der Lichtquelle angezogen wird.

9. Beleuchtungssystem gemäss Anspruch 8, wobei der magnetisierbare Materialanteil in der Buchse der Lichtquelle (4) magnetisiert ist, und/oder
wobei der magnetisierbare Materialanteil und/oder der magnetisierte Materialanteil und/oder der magnetisch anziehbare Materialanteil in der Buchse der Lichtquelle (4) partikelförmig ist und in eine Kunststoffmatrix eingebettet ist, die vorzugsweise durch Spritzgiessen oder Extrudieren herstellbar ist wobei die Partikel während des Spritzgiessens bzw. des Extrudierens einbettbar sind, und wobei der magnetisierbare Materialanteil insbesondere während des Spritzgiessens oder während des Extrudierens oder nach dem Spritzgiessen bzw. durch Anlegen eines Magnetfeldes magnetisiert wird, und/oder
wobei der magnetisierbare Materialanteil oder der magnetisierte Materialanteil im Bereich des distalen Endes (6) der Buchse der Lichtquelle (4), insbesondere in der weiteren Anschlagsfläche (11) der Buchse der Lichtquelle (4), angeordnet ist, und/oder
wobei der magnetisierte Materialanteil als mindestens ein Magnet ausgebildet ist.

10. Beleuchtungssystem gemäss einem der Ansprüche 7 bis 9, wobei die Buchse der Lichtquelle (4) einen im Wesentlichen zylinderförmigen Körper (19) aufweist welcher im Inneren einen Hohlraum (20) definiert,
wobei der Hohlraum (20) in eine Eintrittsöffnung (21) am proximalen Ende (5) und in eine Austrittsöffnung (22) am distalen Ende (6) der Buchse der Lichtquelle (4) mündet, und
wobei sich der Lichtleiter (7) durch die Austrittsöffnung (22) via den Hohlraum (20) zumindest teilweise durch die Eintrittsöffnung (21) erstreckt.

11. Beleuchtungssystem gemäss Anspruch 10, wobei am proximalen Ende (5) der Buchse der Lichtquelle (4) ein Justierflansch (24) angeordnet ist, dessen Durchmesser (DJ) grösser als der Durchmesser (DB) des im Wesentlichen zylinderförmigen Körpers (19) der Buchse der Lichtquelle ist.

12. Beleuchtungssystem gemäss Anspruch 11, wobei der Justierflansch (24) im Querschnitt im Wesentlichen T-förmig ausgebildet ist und sich durch die Eintrittsöffnung (21) der Buchse der Lichtquelle (4) zumindest teilweise in den Hohlraum (20) der Buchse der Lichtquelle (4) hinein erstreckt, und
wobei sich eine durchgehende Öffnung (25) mittig durch den Justierflansch (24) hindurch erstreckt, durch welche Öffnung (25) der Lichtleiter (7) in den Hohlraum (20) der Buchse der Lichtquelle (4) einführbar ist.

13. Beleuchtungssystem gemäss einem der Ansprüche 7 bis 12, wobei eine magnetische Zugkraft zwischen dem Stecker des Lichtinstruments (1) und der Buchse der Lichtquelle (4) zwischen 0.1 N und 5 N, vorzugsweise zwischen 0.5 N und 2 N beträgt, oder
wobei die magnetische Zugkraft gleichviel oder mehr als 2 N beträgt, und/oder
wobei der Stecker des Lichtinstruments (1) und die Buchse der Lichtquelle (4) in einen Abstand (d) zwischen 1 mm und 30 mm, vorzugsweise zwischen 5 mm und 20 mm magnetisch anziehbar ausgebildet sind, oder
wobei der Stecker des Lichtinstruments (1) und die Buchse der Lichtquelle (4) über einen Abstand (d) von mindestens 10 mm, vorzugsweise von mindestens 20 mm magnetisch anziehbar ausgebildet sind.

## Claims

1. Ophthalmic light instrument (33), comprising
a plug of the light instrument (1), having an abutment surface (23); and
a light guide (7), mounted on the plug of the light instrument (1), for guiding light (10) to a surgical site in the eye, wherein the light guide (7) has a proximal end (8) for coupling of light (10) from a light source (12) and a distal end (37) for emission of light (10),
wherein the plug of the light instrument (1) is configured to be magnetically attractable or attractive and can be positioned by means of its abutment surface (23) with respect to the light source and can be connected releasably to the light source,
wherein the plug of the light instrument (1) comprises a magnetisable material component or a magnetised material component,
wherein the magnetisable material component in the plug of the light instrument (1) is magnetised, and
wherein the magnetisable material component and/or the magnetised material component consists of a ferrimagnetic material component, and/or
wherein the magnetisable material component and/or the magnetised material component consists of a ferromagnetic material component,
**characterized in that** the magnetisable material component and/or the magnetised material component is particulate and is embedded in a plastic matrix.

2. Light instrument according to claim 1, wherein the plastic matrix is produced by injection moulding or extrusion, wherein the particles can be embedded during the injection moulding or the extrusion, and wherein the magnetisable material component is in particular magnetised during the injection moulding or during the extrusion or after the injection moulding, respectively by application of a magnetic field.

3. Light instrument according to anyone of the preceding claims, wherein the plug of the light instrument (1) fully consists of the magnetisable material component or of the magnetised material component, or
wherein the magnetisable material component or the magnetised material component is arranged in the region of the proximal end (2) of the plug of the light instrument (1), particularly in the abutment surface (23) of the plug of the light instrument (1).

4. Light instrument according to anyone of the preceding claims, wherein the plug of the light instrument (1) comprises an essentially cylindrical body (15), which internally defines a cavity (16),
wherein the cavity (16) opens into an entry orifice (17) at the proximal end (2) and into an exit orifice (18) at the distal end (3) of the plug of the light instrument (1), and
wherein the light guide (7) extends through the entry orifice (17), via the cavity (16), and through the exit orifice (18).

5. Light instrument according to Claim 4, wherein the abutment surface (23) at the proximal end (2) of the plug of the light instrument (1) is configured as a flange, the diameter (DF) of which is greater than or equal to the diameter (DS) of the essentially cylindrical body (15) of the plug of the light instrument (1).

6. Light instrument according to anyone of the preceding claims, wherein the light guide (7) is firmly mounted on or in the plug (1), wherein the light guide (7) is firmly mounted in the plug (1), particularly by means of a guide tube (13) which extends through the plug (1).

7. Ophthalmic illumination system (32) comprising a light instrument according to anyone of the preceding Claims 1 to 6 and a socket of the light source (4), having a further abutment surface (11), **characterized in that**
the socket of the light source (4) is configured to be magnetically attractable or attractive, and **in that** the abutment surface (23) of the plug comes in contact with the further abutment surface (11) of the socket, so that the light guide (7) can be positioned with respect to the light source in such a way, and can be connected releasably to the plug of the light instrument (1) in such a way, that the proximal end (8) of the light guide (7) comes to lie at the focal point of the light source, so that coupling of light (10) takes place at the focal point (14) of the light source (12).

8. Illumination system according to Claim 7, wherein the socket of the light source (4) comprises a magnetisable material component or a magnetised material component or a magnetically attractable material component, and wherein the material component of the socket of the light source is selected in such a way that the plug of the light instrument is magnetically attracted towards the socket of the light source.

9. Illumination system according to Claim 8, wherein the magnetisable material component in the socket of the light source (4) is magnetised, and/or
wherein the magnetisable material component and/or the magnetised material component and/or the magnetically attractable material component in the socket of the light source (4) is particulate and is embedded in a plastic matrix, which can preferably be produced by injection moulding or extrusion, wherein the particles can be embedded during the injection moulding or the extrusion, and wherein the magnetisable material component is in particular magnetised during the injection moulding or during the extrusion or after the injection moulding, respectively by application of a magnetic field, and/or
wherein the magnetisable material component or the magnetised material component is arranged in the region of the distal end (6) of the socket of the light source (4), particularly in the further abutment surface (11) of the socket of the light source (4), and/or
wherein the magnetised material component is configured as at least one magnet.

10. Illumination system according to anyone of Claims 7 to 9, wherein the socket of the light source (4) comprises an essentially cylindrical body (19), which internally defines a cavity (20),
wherein the cavity (20) opens into an entry orifice (21) at the proximal end (5) and into an exit orifice (22) at the distal end (6) of the socket of the light source (4), and
wherein the light guide (7) extends through the exit orifice (22), via the cavity (20), and at least partially through the entry orifice (21).

11. Illumination system according to Claim 10, wherein an adjustment flange (24), the diameter (DJ) of which is greater than the diameter (DB) of the essentially cylindrical body (19) of the socket of the light source, is arranged at the proximal end (5) of the socket of the light source (4).

12. Illumination system according to Claim 11, wherein the adjustment flange (24) is configured essentially with a T-shape in cross section and extends through the entry orifice (21) of the socket of the light source (4) at least partially into the cavity (20) of the socket of the light source (4), and
wherein a through-orifice (25) extends centrally through the adjustment flange (24), through which orifice (25) the light guide (7) can be inserted into the cavity (20) of the socket of the light source (4).

13. Illumination system according to anyone of Claims 7 to 12, wherein a magnetic pulling force between the plug of the light instrument (1) and the socket of the light source (4) lies between 0.1 N und 5 N, preferably between 0.5 N and 2 N, or
wherein the magnetic pulling force is equal to or greater than 2 N, and/or
wherein the plug of the light instrument (1) and the socket of the light source (4) are configured to be magnetically attractable at a distance (d) of between 1 mm and 30 mm, preferably between 5 mm and 20 mm, or
wherein the plug of the light instrument (1) and the socket of the light source (4) are configured to be magnetically attractable over a distance (d) of at least 10 mm, preferably at least 20 mm.

## Revendications

1. Un instrument à lumière ophtalmique (33) comprenant
une fiche de l'instrument à lumière (1) ayant une surface d'arrêt (23) ; et
un guide de lumière (7) monté sur la fiche de l'instrument à lumière (1) pour guider de la lumière (10) vers un site chirurgical dans l'oeil, le guide de lumière (7) ayant une extrémité proximale (8) pour coupler de la lumière (10) à partir d'une source de lumière (12) et une extrémité distale (37) pour émettre de la lumière (10),
la fiche de l'instrument à lumière (1) étant conçue pour être magnétiquement attirante respectivement attirable et pouvant être positionnée par l'intermédiaire sa face de d'arrêt (23) par rapport à la source de lumière et pouvant être reliée de manière amovible à la source de lumière,
la fiche de l'instrument à lumière (1) ayant une partie de matériau magnétisable ou une partie de matériau magnétisé,
la partie de matériau magnétisable dans la fiche de l'instrument à lumière (1) étant magnétisée, et
la partie de matériau magnétisable et/ou la partie de matériau magnétisé étant constituée d'une partie de matériau ferrimagnétique, et/ou
la partie de matériau magnétisable et/ou la partie de matériau magnétisé étant constituée d'une partie de matériau ferromagnétique,
**caractérisé en ce que** la partie de matériau magnétisable et/ou la partie de matériau magnétisé est en forme de particules et intégrée dans une matrice de matériau plastique.

2. Instrument à lumière selon la revendication 1, où la matrice en matériau plastique est réalisable par injection moulage ou extrusion, où les particules peuvent être intégrées lors de l'injection moulage respectivement de l'extrusion, et où la partie de matériau magnétisable est magnétisé en particulier lors de l'injection moulage ou lors de l'extrusion ou après l'injection moulage respectivement en appliquant un champ magnétique.

3. Instrument à lumière selon une quelconque des revendications précédentes, où la fiche de l'instrument à lumière (1) est entièrement constituée de partie de matériau magnétisable ou de partie de matériau magnétisé, où la partie de matériau magnétisable ou la partie de matériau magnétisé est disposée dans la région de l'extrémité proximale (2) de la fiche de l'instrument à lumière (1), en particulier dans la surface d'arrêt (23) de la fiche de l'instrument à lumière (1).

4. Instrument à lumière selon une des revendications précédentes, où la fiche de l'instrument à lumière (1) présente un corps sensiblement en forme de cylindre (15), lequel définit une cavité (16) à l'intérieur,
où la cavité (16) débouche dans un ouverture d'entrée (17) au niveau de l'extrémité proximale (2) et dans un ouverture de sortie (18) au niveau de l'extrémité distale (3) de la fiche de l'instrument à lumière (1), et
où le guide de lumière (7) s'étend à travers de l'ouverture d'entrée (17) et à travers l'ouverture de sortie (18) via la cavité (16).

5. Instrument à lumière selon la revendication 4, où la surface d'arrêt (23) est réalisée au niveau de l'extrémité proximale (2) de la fiche de l'instrument à lumière (1) en tant que bride, dont le diamètre (DF) est supérieur ou égal au diamètre (DS) du corps sensiblement en forme cylindre (15) de la fiche de l'instrument à lumière (1).

6. Instrument à lumière selon une des revendications précédentes, où le guide de lumière (7) est monté de manière fixe sur, respectivement dans, la fiche (1), où le guide de lumière (7) est monté de manière fixe dans la fiche (1) en particulier au moyen d'une conduite de guidage (13) s'étendant à travers la fiche (1).

7. Un système d'illumination ophtalmique (32) comprenant un instrument à lumière selon une des revendications 1 à 6 et une prise femelle de la source de lumière (4), avec une surface d'arrêt supplémentaire (11), **caractérisé en ce que**, la prise femelle de la source de lumière (4) est pour être magnétiquement attirante respectivement attirable et que la surface d'arrêt de la fiche (23) vient en contact avec la surface d'arrêt supplémentaire de la prise femelle (11), de sorte que le guide de lumière (7) peut être positionné de telle sorte par rapport à la source de lumière et peut être relié de manière amovible à la fiche de l'instrument à lumière (1) que l'extrémité proximale (8) du guide de lumière (7)) aboutit au niveau du point focal de la source de lumière, de sorte que un découplage de la lumière (10) au niveau du point focal (14) de la source de lumière (10) est réalisé.

8. Système d'illumination selon la revendication 7, où la prise femelle de la source de lumière (4) présente une partie de matériau magnétisable ou une partie de matériau magnétisé ou une partie de matériau pouvant être magnétiquement attiré, et où la partie de matériau de la prise femelle de la source de lumière est choisi de sorte que la fiche de l'instrument de lumière est attirée de manière magnétique vers la prise femelle de la source de lumière.

9. Système d'illumination selon la revendication 8, où la partie de matériau magnétisable dans la prise femelle de la source de lumière (4) est magnétisé, et / ou où la partie de matériau magnétisable et / ou la partie de matériau magnétisé et / ou la partie de matériau pouvant être magnétiquement attiré dans la prise femelle de la source de lumière (4) est en forme de particules est intégrée dans une matrice de matériau plastique, préférablement réalisable par injection moulage ou extrusion ou les particules sont intégrables lors de l'injection moulage respectivement de l'extrusion, et où la partie de matériau pouvant être magnétisée est magnétisée en particulier lors de l'injection moulage ou lors de l'extrusion ou après l'injection moulage, respectivement par application d'un champ magnétique, et / ou
où la partie de matériau magnétisable ou la partie de matériau magnétisé est disposée dans la région de l'extrémité distale (6) de la prise femelle de la source de lumière (4), en particulier dans la surface d'arrêt (11) supplémentaire de la prise femelle de la source de lumière (4), et / ou
où la partie de matériau magnétisé est réalisée en tant que au moins un aimant.

10. Système d'illumination selon une des revendications 7 à 9, où la prise femelle de la source de lumière (4) présente un corps (19) sensiblement en forme de cylindre lequel définit une cavité (20) à l'intérieur,
où la cavité (20) débouche dans un ouverture d'entrée (21) au niveau de l'extrémité proximale (5) et dans un ouverture de sortie (22) au niveau d'une extrémité distale (6) de la prise femelle de la source de lumière (4), et
où le guide de lumière (7) s'étend à travers de l'ouverture de sortie (22) et au moins partiellement à travers l'ouverture d'entrée (21) via la cavité (20).

11. Système d'illumination selon la revendication 10, où au niveau de l'extrémité proximale (5) de la prise femelle de la source de lumière (4) une bride d'ajustement (24) est disposée, dont le diamètre (DJ) est supérieur au diamètre (DB) du corps sensiblement en forme de cylindre (19) de prise femelle de la source de lumière (4).

12. Système d'illumination selon la revendication 11, où la bride d'ajustement (24) est sensiblement réalisé en forme de T en section transversale et s'étend au moins partiellement à travers l'ouverture (21) de la prise femelle de la source de lumière (4) au moins partiellement dans la cavité (20) de la prise femelle de la source de lumière (4), et
où un ouverture (25) traversante s'étend à travers la bride d'ajustement (24) de manière centrale, à travers laquelle ouverture (25) le guide de lumière (7) peut être inséré dans la cavité (20) de la prise femelle de la source lumineuse (4).

13. Système d'illumination selon une des revendications 7 à 12, où une force de traction magnétique entre la fiche de l'instrument à lumière (1) et de la prise femelle de la source de lumière (4) est entre 0,1 N et 5 N, préférablement entre 0,5 N et 2 N, ou
où la force de traction magnétique est égale ou supérieure à 2 N, et / ou
où la fiche de l'instrument à lumière (1) et la prise femelle de la source de lumière (4) sont réalisées de manière à être magnétiquement attirables à une distance (d) entre 1 mm et 30 mm, préférablement entre 5 mm et 20 mm, ou
où la fiche de l'instrument à lumière (1) et la prise femelle de la source de lumière (4) sont réalisées de manière magnétiquement attirables à une distance (d) d'au moins 10 mm, préférablement d'au moins 20 mm.
